(19) Europäisches Patentamt / European Patent Office / Office européen des brevets

(11) **EP 2 546 631 B1**

(12) **EUROPEAN PATENT SPECIFICATION**

(45) Date of publication and mention
of the grant of the patent:
**09.04.2014 Bulletin 2014/15**

(51) Int Cl.:
***G01N 19/02*** (2006.01)

(21) Application number: **12157364.6**

(22) Date of filing: **28.02.2012**

(54) **Device and method to perform grip tests**

Anordnung und Verfahren zum Durchführen von Rutschtests

Dispositif et méthode pour effectuer des tests d'adhérence

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.07.2011 IT VR20110149**

(43) Date of publication of application:
**16.01.2013 Bulletin 2013/03**

(73) Proprietor: **Vibram S.p.A.
21041 Albizzate (Varese) (IT)**

(72) Inventors:
• **Bramani, Marco
21041 Albizzate (Varese) (IT)**
• **Sacchi, Massimiliano
21041 Albizzate (Varese) (IT)**
• **Faccinetto, Nicola
21041 Albizzate (Varese) (IT)**

(74) Representative: **Feltrinelli, Secondo Andrea
APTA S.r.l.
Patent Department
Via Ca' di Cozzi, 41
37124 Verona (IT)**

(56) References cited:
**WO-A2-2009/077733     US-A- 4 187 714**

• **Graeme Hunwin ET AL: "A study of the effect of modifying the European Standard mechanical slip resistance test for footwear", Health and Safety Executive 06/10, 1 January 2010 (2010-01-01), pages 1-23, XP055025940, Retrieved from the Internet: URL:http://www.hse.gov.uk/research/rrpdf/r r801.pdf [retrieved on 2012-04-30]**
• **In-Ju Kim ET AL: "Research on Slip Resistance Measurements - A New challenge", Industrial Health, 1 January 2008 (2008-01-01), pages 66-76, XP055025938, Retrieved from the Internet: URL: http://www.jniosh.go.jp/en/indu_hel/pd f/IH_ 46_1_66.pdf [retrieved on 2012-04-30]**

## Description

### TECHNICAL FIELD OF THE INVENTION

The present invention refers to a device for carrying out grip tests, in particular between shoe soles and different test surfaces, and a method for carrying out grip tests through such a device.

### STATE OF THE ART

In the prior art there are machines available on the market for determining resistance to slipping, i.e. loss of grip, of a material with respect to a test surface. See e.g. US-A-4187714 or WO-A-2009/077733. Such resistance to slipping is evaluated through the measurement of the friction coefficient of the test surface itself. A further example is given by the Brungraber Mark II machine, in which a sliding block, slidably fastened to a frame, is pressed against the ground and, at the same time, pushed in the direction parallel to the ground itself. The friction coefficient of a test surface is determined by verifying the thrust stress, induced on the sliding block, necessary to cause the sliding block to slide on the test surface itself.

Although such machines allow the static friction coefficient of a test surface to be determined, nevertheless they do not make it possible to characterise, for example, the ability of a shoe sole to grip in normal use of the shoe itself, without slipping, on a test surface.

Such machines do not make it possible to significantly reproduce the real contact between the shoe sole, subjected to the weight of the human body, and a surface, in dry or wet conditions.

### PURPOSES OF THE INVENTION

A purpose of the present invention is to improve the state of the art.

Another purpose of the present invention is to propose a device for carrying out grip tests, in particular between a shoe sole and different test surfaces, which makes it possible to evaluate the gripping capability of a sole with respect to different test surfaces.

In accordance with an aspect of the present invention a device according to claim 1 is foreseen.

A further purpose of the present invention is to present a method for carrying out grip tests. According to an aspect of the present invention a method for carrying out a grip test as specified in claim 11 is foreseen.

The dependent claims refer to preferred and advantageous embodiments of the invention.

### BRIEF DESCRIPTION OF THE DRAWINGS

Further characteristics and advantages of the present invention will become clearer from the detailed description of a preferred, but not exclusive, embodiment of a device for carrying out grip tests, illustrated for indicating but not limiting purposes, in the attached tables of drawings, in which:

figure 1 is a schematic perspective view of the device;
figure 2 is a schematic perspective view of another embodiment of the device;
figure 3 is a simplified chart for carrying out a grip test;
figure 4 is a simplified chart relative to the calculation of some parameters of a grip test.

### EMBODIMENTS OF THE INVENTION.

With reference to the attached figures, reference numeral 1 schematically indicates a device for carrying out grip tests.

Such a device 1 allows an operator 14, wearing the shoes with the soles 15 under testing, to carry out grip tests between the soles 15 and different test surfaces, each having different surface characteristics, for example surface roughness, material from which such a surface is made, configuration of the surface, etcetera.

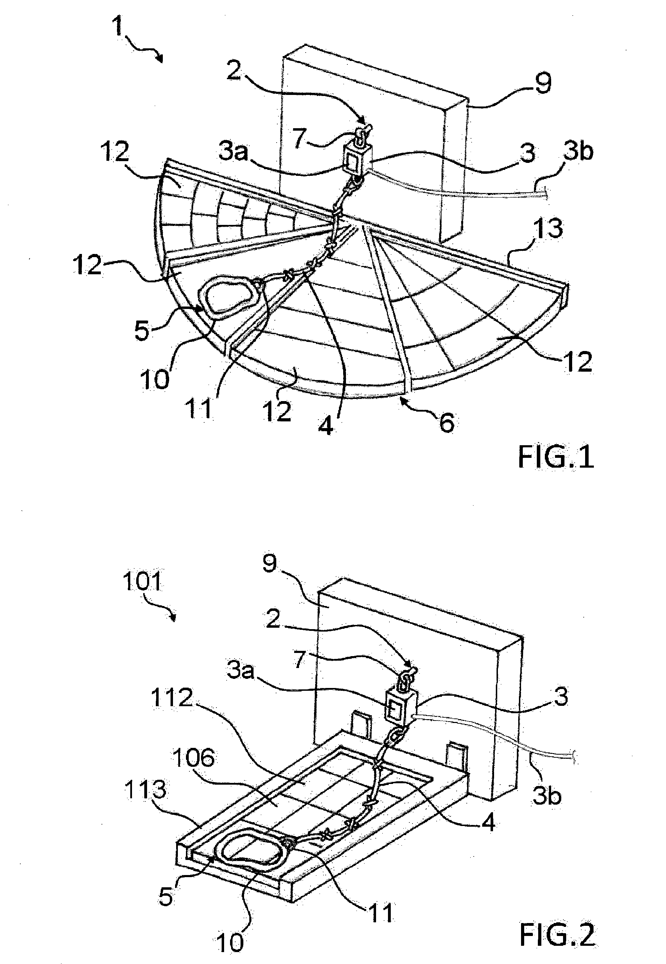

In reference to the embodiment of figure 1, the device 1 for carrying out grip tests of a shoe sole 15 worn by an operator 14 mainly comprises at least one fastening element 2, a measuring device 3 connected to the fastening element 2, means 5 for holding the operator 14, traction means 4 fixedly connected at a first end to said measuring device 3 and at a second end to said holding means 5, in which said operator 14 is positioned on a test surface 6 to carry out a grip test.

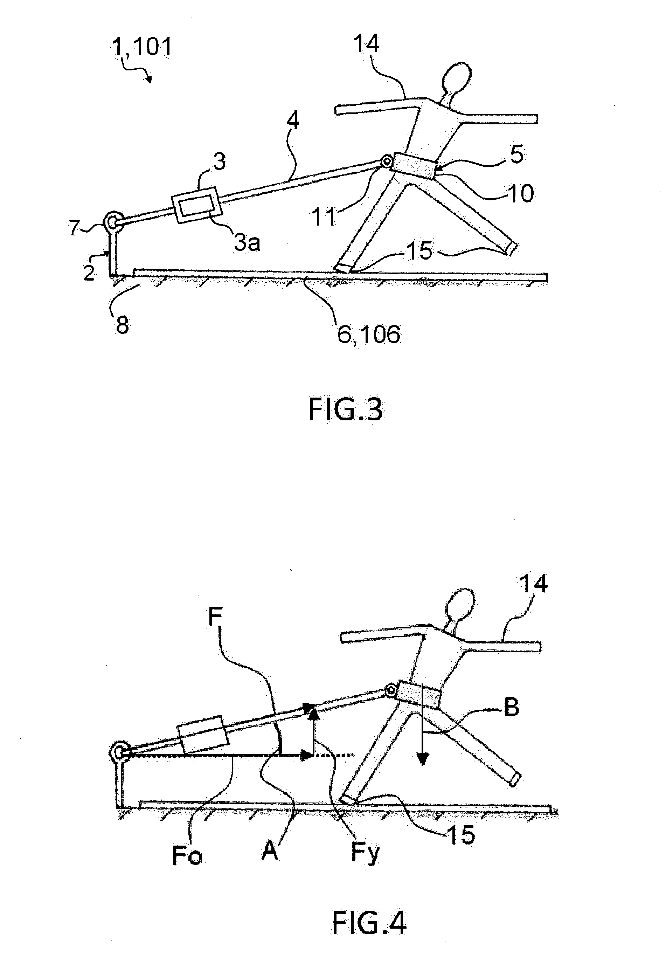

The fastening element 2, at a first end, is fixedly connected to a wall, or a frame, or in general to an immobile structure and, at a second end opposite the first, it is connected to the measuring device 3. According to the diagram shown in figure 3, the fastening element 2 can be fixedly connected, at an end thereof, to the ground 8.

In a further embodiment, as illustrated in figure 1 and 2, the fastening element 2 can be fixedly connected, at an end thereof, to a vertical wall 9, actually being immobile.

The fastening element 2, for example an eyebolt, is equipped with a cylindrical elongated element, at one end of which an eyelet 7 is foreseen. As shown in the figures, through the eyelet 7 it is possible to connect the measuring device 3 to said fastening element 2.

The measuring device 3, for example an electronic dynamometer equipped with a load cell, or a mechanical dynamometer, is connected at one end to the fastening element 2 and, at the opposite end, to the traction means 4.

The measuring device 3 can also comprise a viewer or display 3a, on which it is possible to display the value of the force with which the traction means 4 are placed under tension by an operator 14 while the grip test is being performed.

Moreover, the measuring device 3 can comprise an electronic data storage device for acquiring and recording the tension values applied during the grip test. Furthermore, the measuring device can comprise an electronic USB port, or similar elements, to which it is possible to connect a cable 3b to allow the transmission of data to a computer not shown in the figures.

The traction means 4 can be made through a cable element and/or any rigid or flexible element suitable for the purpose.

The length of the traction means 4 can be varied, according to the needs of the operator 14 while each grip test is being performed.

The holding means 5 allow an operator 14 to safely attach himself to the device 1.

Moreover, the holding means 5 allow the operator 14 to exert a traction force on the fastening element 2 through the traction means 4.

The holding means 5 comprise a safety harness 10, suitably adjustable, to be able to be worn by an operator 14, as illustrated in figure 3 and 4.

Such a safety harness 10 comprises at least one connection element 11 for fastening the harness 10 itself to the traction means 4. The connection means 11 can be made through a ring, or a spring catch, or a similar element suitable for the purpose, which makes it possible to connect and/or separate the harness 10 to/from the traction means 4 in a practical and quick manner, and to ensure a stable and safe connection while the grip test is being carried out.

The test surface 6, according to the embodiment of figure 1, can have a substantially semi-circular configuration and comprise a series of areas 12.

Each area 12 can have different characteristics from one another, like, for example, surface roughness and/or type of material and/or configuration of the surface, etcetera.

The areas 12, in a particular embodiment, can be positioned beside one another, defining portions of the test surface 6 substantially similar to circular sectors, as shown in figure 1.

In a further embodiment, not shown in the figures, the test surface can have a substantially circular shape, divided in areas similar to circular sectors and positioned beside one another.

It is possible to foresee further arrangements of the areas 12 inside the test surface 6, all covered by the scope of protection of the invention.

A further embodiment of the present invention is shown in figure 2. For greater clarity, the same reference numerals are used for parts that are the same as those already described earlier and that have the same functions.

The device 101, as shown in figure 2, comprises a test surface 106 made from a material, the surface roughness, the type of material, the configuration of the surface of which, etcetera, are known.

The test surface 106 can be replaced with another test surface, having different characteristics, such as the material, the surface roughness, the configuration of the surface, etcetera.

The roughness of the test surface 6, 106 is measured in the contact area between the sole to be tested and the surface 6, 106 itself.

Moreover, the test surface 6, 106 can be wetted, with different types of fluids, like for example water and/or oil and/or paraffin, etcetera, thus varying the degree of grip of the test surface 6, 106 itself. By doing this, it is possible to reproduce the conditions of use of the soles to be tested, in different environments, like for example industrial kitchen floors and/or workshop floors and/or industrial locations and/or workplaces, and other, in general.

The test surface 6, 106 can be fixedly connected to the ground or, in general, made immobile with respect to the fastening element 2, to avoid relative sliding, while the grip test is being carried out, between the fastening element 2 and the test surface 6, 106 itself.

Such relative sliding would not allow the grip of the sole under testing to be evaluated with respect to the test surface 6, 106.

The device 1, 101 can also comprise a horizontal frame 13, 113 on which the test surface 6, 106 is to be arranged and fixedly connected, as shown in figure 1 and 2.

The horizontal frame 13, 113, in a preferred but not limiting embodiment, is fixedly connected to the ground in order to avoid relative sliding, while the grip test is being carried out, between the fastening element 2 and the test surface 6, 106 itself. Moreover, in a further possible embodiment of the device 1, not shown in the figures, the fastening element 2 could be fixedly connected directly to the horizontal frame 13, 113 for example, purely for indicating purposes, through welding.

The operation of the present invention relative to the figures is as follows.

An operator 14, after having put on the shoes with the sole 15 under testing, the characteristics of which are not known to him, wears the harness 10. Then, the operator 14 fastens the harness 10 to the traction means 4, through the connection element 11. The operator 14 positions himself on the test surface 6, 106 in upright position, and, if necessary, modifies the length of the traction means 4, in order to place them slightly under tension with respect to the fastening element 2.

Then the operator 14 carries out a series of predefined movements, moving the barycentre of his own body, to induce a traction stress along the traction means 4, until there is a loss of grip of the sole, on which the operator 14 stands, with respect to the test surface 6, 106.

The measuring device 3, while the grip test is being carried out, records the instantaneous traction force exerted by the operator 14 through the traction means 4, to allow the gripping capability of the soles 15 to be determined with respect to the test surface 6. Through the following relationships, relative to the forces that are generated while a grip test is being carried out, a friction coefficient CoF is determined, with which it is possible to quantify the grip of the material constituting the soles 15 under testing.

The friction coefficient CoF is the ratio between the horizontal component Fo of the traction force F, generated by the operator 14, and the vertical force Fv. The latter force comprises both the component due to the weight Bw of the operator 14 and the vertical component Fy of the traction force F.

The friction coefficient CoF, according to the diagram shown in figure 4, is thus expressed by the following relationship:

$$CoF = Fo/Fv$$

In which:

$$Fo = FcosA$$

and

$$Fv = B - FsenA = B - Fy$$

Where A is the angle of inclination of the traction means 4 with respect to horizontal, i.e. the ground, and F is the tension applied by the operator 14 and detected by the measuring means 3.

The present invention also refers to a method for carrying out a grip test by using the device 1, 101 described above.

Such a method for carrying out the grip test mainly comprises three steps:

> a first step of preparing the test surface 6, 106 and the shoe sole 15 to be tested;
> a second step in which a series of grip tests are carried out to allow an operator 14 wearing the shoes with the soles 15 to be tested to familiarise himself with the device 1, 101 and with the movements to be carried out during the grip test;
> a third step in which the grip test between the soles 15 of the shoes worn by an operator 14, and at least one test surface 6, 106 of the device 1, 101 is carried out, and the results are recorded.

The first step of the method for carrying out a grip test comprises the preparation of the test surface 6, 106, and of the shoe soles 15 to be tested, divided into the following steps:

> cleaning the test surface 6, 106 with an ethanol and water based solution, in which the fraction by mass of ethanol in the water is equal to 50% with variations of plus or minus 5%;

drying the test surface 6, 106 placed at room temperature;
cleaning the sole 15 of the shoe to be tested with an ethanol and water based solution, in which the fraction by mass of ethanol in the water is equal to 50% with variations of plus or minus 5%
drying the sole 15 of the shoe to be tested placed at room temperature;
abrading the sole 15 of the shoe carried out by delicately pressing sandpaper made from silicon carbide or similar against the sole 15.

The abrasion intends to make the appearance of the sole 15 to be placed in contact with the test surface 6, 106 uniform, without substantially modifying its configuration or its possible tread.

Moreover, it is possible to remove possible debris through compressed air jets or soft, dry and clean brushes.

The second step of the method for carrying out a skid test comprises:

> the operator 14 who wears a pair of shoes with the sole 15 to be tested, after having positioned himself on the test surface 6, 106, carrying out a series of movements suitable for placing the traction means 4 under tension.
> Carrying out such a series of movements allows the operator 14 to gain confidence with the device 1, 101 and to take up a standard and repeatable movement dynamic in order to reduce the variability of the grip test due to the operator himself.

The third step of the method for carrying out a grip test comprises the operator 14 carrying out a series of movements:

> making the operator 14 take up a lateral position on the test surface 6, 106. The operator 14 positions his feet in a direction perpendicular to the traction means 4, with the lower limbs straightened;
> the operator 14 bending the leg positioned at the rear with respect to the fastening element 2, until it is completely raised from the test surface 6, 106. During this step the operator 14 keeps in static equilibrium on the leg resting on the test surface 6, 106;
> bending the side of the trunk of the operator 14 in the opposite direction to the fastening element 2, thus determining the progressive increase in tension applied to the traction means 4. The test continues until there is a loss of grip, and therefore slipping, of the sole 15 of the shoe worn by the operator 14, with respect to the test surface 6, 106.

The method according to the present invention also comprises the following steps:

> checking that the operator 14 is correctly positioned on the test surface 6, 106 while the grip test is being carried out;

verifying that the sole 15 of the shoe is placed completely in contact with the test surface 6, 106;

verifying whether the loss of grip of the sole 15 with respect to the test surface 6, 106 occurs within a time period of between two and four seconds from the start of the grip test;

verifying whether the data measured by the measuring device 3, while at least three grip tests were carried out, are congruent, i.e. the value of each test does not significantly differ from the average value of the three tests.

The device 1, 101 according to the present invention makes it possible to carry out grip tests of a shoe sole 15, worn by an operator 14, with respect to different test surfaces 6, 106 in order to completely characterise the gripping capability of the sole 15 itself in a simple, effective and low-cost solution. It is also possible to analyse the grip of soles consisting of different materials with respect to various test surfaces 6, 106 by simulating conditions of use analogous to those encountered in a normal daily routine.

The grip of the sole 15 can be verified both in dry and in wet conditions.

Furthermore, it is possible to modify the test surface 6, 106 on which the grip test is to be carried out in a quick and simple manner.

The invention thus conceived can undergo numerous modifications and variants, all of which are covered by the inventive concept.

Moreover, all of the details can be replaced by other technically equivalent elements. In practice, the materials used, as well as the contingent shapes and sizes, can be whatever according to the requirements without for this reason departing from the scope of protection of the following claims.

## Claims

1.  Device (1) to perform grip tests of a sole (15) for footwear, said footwear with said footwear sole (15) to be tested being worn by an operator (14), said device (1) comprising at least a fastening element (2), a measuring device (3) connected to said fastening element (2), means (5) for holding associable to said operator (14) who wears said footwear, a traction means (4) constrained at a first end to said measuring device (3) and at a second end to said holding means (5), **characterised in that** it comprises a test surface (6, 106) for performing a grip test of said footwear sole (15), on said test surface (6, 106) being positionable said operator (14).

2.  Device according to claim 1, wherein said fastening element (2) is constrained stationary with respect to said test surface (6, 106), for example being constrained, at an end thereof, to a ground (8) or to a vertical wall (9).

3.  Device according to claim 1 or 2, wherein said fastening element (2) comprises an eyelet (7) for being connected to said measuring device (3).

4.  Device according to any one of the previous claims, wherein said measuring device (3) comprises an electronic dynamometer equipped with a load cell and/or a mechanical dynamometer.

5.  Device according to any one of the previous claims, wherein said traction means (4) comprises a rope element and/or any rigid or flexible element suitable for the purpose.

6.  Device according to any one of the previous claims, wherein said traction means (4) has a variable length.

7.  Device according to any one of the previous claims, wherein said holding means (5) comprise a safety harness (10) and at least a connecting element (11), made from a ring and/or a spring catch and/or a similar element, for connecting said harness (10) to said traction means (4).

8.  Device according to any one of the previous claims, wherein said test surface (6, 106) is placed on a horizontal frame (13, 113) constrained stationary to the ground.

9.  Device according to any one of the previous claims, wherein said test surface (6) has a substantially semicircular and/or circular shape.

10. Device according to any one of the previous claims from 1 to 8, wherein said test surface (6) has a substantially semicircular or circular shape and comprises several areas (12) radially placed one next to the other, each having its own features such as surface roughness and/or type of material and/or surface finish, etc..

11. Method for performing a grip test for footwear soles comprising the steps of:

providing a device (1, 101) including at least a fastening element (2), a measuring device (3), a means (5) for holding an operator (14), and at least a test surface (6, 106)
**characterised in that** it comprises the following steps:

preparing said test surface (6, 106) and a footwear sole (15) to be tested;

performing a grip test of said footwear soles (15) on at least one of said test surface (6, 106) of the device (1), said footwear, provided with said footwear sole (15), being worn by said operator (14);

recording parameters of said grip test and data provided by said measuring device (3).

12. Method according to claim 11, comprising spraying said test surface (6, 106) with different substances, for example water and/or oils and/or paraffins, etc..

13. Method according to claim 11 or 12, comprising cleaning said test surface (6, 106) with water, for example mains water, and/or an ethanol solution.

14. Method according to any one of claims from 11 to 13, comprising cleaning said soles to be tested with a solution of ethanol and water and/or by abrasion carried out with abrasive paper without substantially modifying said soles, in particular a possible outsole of said soles, and/or by compressed air jets or soft brushes.

15. Method according to any one of claims 11 to 14, comprising:

making said operator (14) take on a lateral position on said test surface (6, 106), said operator (14) placing his/her feet in a direction orthogonal with respect to said traction means (4);

making said operator (14) bend a leg, said leg being placed backward with respect to said fastening element (2), up to completely raising said leg from said test surface (6, 106), said operator (14) balancing in a static way on said resting leg;

making said operator (14) bend laterally in a direction opposite to said fastening element (2), until said sole (15) loses grip with respect to said test surface (6, 106).

16. Method according to any one of claims 11 to 15, comprising:

making sure that said operator (14) is properly placed on said test surface (6, 106) during the performing of said grip test;

making sure that said sole (15) is placed completely in contact with said test surface (6, 106);

making sure that said sole (15) loses grip with respect to said test surface (6, 106) within a time period ranging from two to four seconds from the beginning of said grip test.

17. Method according to any one of claims 11 to 16, comprising:

analyzing data provided by said measuring device (3), during said grip tests, in order to verify the gripping power of said footwear sole (15) with respect to said test surfaces (6, 106).

**Patentansprüche**

1. Vorrichtung (1) zum Ausführen von Prüfungen der Haftreibung einer Sohle (15) für Schuhwerk, wobei dieses Schuhwerk mit dieser zu prüfenden Schuhsohle (15) von einem/einer Laboranten/Laborantin (14) getragen wird, wobei diese Vorrichtung (1) mindestens ein Befestigungselement (2), eine mit diesem Befestigungselement (2) verbundene Messeinrichtung (3), Mittel (5) zum Halten, die mit dem/der Laboranten/Laborantin (14), der/die das Schuhwerk trägt, verbunden werden können, und ein Zugmittel (4) umfasst, das an einem ersten Ende an die Messeinrichtung (3) und an einem zweiten Ende an den Haltemitteln (5) gebunden ist, **dadurch gekennzeichnet, dass** sie eine Prüfoberfläche (6, 106) zum Ausführen einer Prüfung der Haftreibung dieser Schuhsohle (15) umfasst, wobei der/die Laborant/Laborantin (14) auf dieser Prüfoberfläche (6, 106) stehen kann.

2. Vorrichtung nach Anspruch 1, wobei das Befestigungselement (2) in Bezug auf die Prüfoberfläche (6, 106) ortsfest gebunden ist, indem es beispielsweise an einem seiner Ende an einem Boden (8) oder an einer senkrechten Wand (9) gebunden ist.

3. Vorrichtung nach Anspruch 1 oder 2, wobei das Befestigungselement (2) eine Öse (7) umfasst, um mit der Messeinrichtung (3) verbunden zu werden.

4. Vorrichtung nach einem der vorhergehenden Ansprüche, wobei die Messeinrichtung (3) einen elektronischen Kraftmesser, der mit einer Kraftmessdose ausgestattet ist, und/oder einen mechanischen Kraftmesser umfasst.

5. Vorrichtung nach einem der vorhergehenden Ansprüche, wobei das Zugmittel (4) ein Seilelement und/oder ein beliebiges zweckdienliches starres oder flexibles Element umfasst.

6. Vorrichtung nach einem der vorhergehenden Ansprüche, wobei das Zugmittel (4) eine variable Länge hat.

7. Vorrichtung nach einem der vorhergehenden Ansprüche, wobei die Haltemittel (5) ein Sicherheitsgeschirr (10) und mindestens ein Verbindungselement (11), das aus einem Ring und/oder einem Karabinerhaken und/oder einem ähnlichen Element zum Verbinden des Geschirrs (10) mit dem Zugmittel (4) besteht, umfassen.

**8.** Vorrichtung nach einem der vorhergehenden Ansprüche, wobei sich die Prüfoberfläche (6, 106) auf einem waagrechten Rahmen (13, 113) befindet, der ortsfest an dem Boden gebunden ist.

**9.** Vorrichtung nach einem der vorhergehenden Ansprüche, wobei die Prüfoberfläche (6) im Wesentlichen halbkreisförmig und/oder kreisförmig ist.

**10.** Vorrichtung nach einem der vorhergehenden Ansprüche von 1 bis 8, wobei die Prüfoberfläche (6) im Wesentlichen halbkreisförmig oder kreisförmig ist und mehrere radial nebeneinander angeordnete Bereiche (12) umfasst, von denen jeder seine eigenen Merkmale wie Oberflächenrauheit und/oder Materialtyp und/oder Oberflächenbeschaffenheit usw. aufweist.

**11.** Verfahren zum Ausführen einer Haftreibungsprüfung für Schuhsohlen, das die folgenden Schritte umfasst:

Bereitstellen einer Vorrichtung (1, 101), die mindestens ein Befestigungselement (2), eine Messeinrichtung (3), ein Mittel (5) zum Halten eines/einer Laboranten/Laborantin (14) und mindestens eine Prüfoberfläche (6, 106) umfasst, **dadurch gekennzeichnet, dass** es die folgenden Schritte umfasst:

Vorbereiten der Prüfoberfläche (6, 106) und einer zu prüfenden Schuhsohle (15);
Ausführen einer Prüfung der Haftreibung von diesen Schuhsohlen (15) auf mindestens einer dieser Prüfoberflächen (6, 106) der Vorrichtung (1), wobei dieses Schuhwerk, das mit der Schuhsohle (15) versehen ist, von dem/der Laboranten/Laborantin (14) getragen wird;
Aufzeichnen von Parametern dieser Haftreibungsprüfung und von Daten, die von der Messeinrichtung (3) bereitgestellt werden.

**12.** Verfahren nach Anspruch 11, welches das Besprühen der Prüfoberfläche (6, 106) mit verschiedenen Stoffen wie beispielsweise Wasser und/oder Öle und/oder Paraffine usw. umfasst.

**13.** Verfahren nach Anspruch 11 oder 12, welches das Reinigen der Prüfoberfläche (6, 106) mit Wasser, beispielsweise Leitungswasser, und/oder einer Ethanollösung umfasst.

**14.** Verfahren nach einem der Ansprüche von 11 bis 13, welches das Reinigen der zu prüfenden Sohlen mit einer Lösung aus Ethanol und Wasser und/oder durch Abschleifen, das mit Schleifpapier durchgeführt wird, ohne diese Sohlen, insbesondere eine etwaige Außensohle dieser Sohlen, wesentlich zu verändern, und/oder mit Druckluftstrahlen oder weichen Bürsten umfasst.

**15.** Verfahren nach einem der Ansprüche 11 bis 14, umfassend:

Veranlassen des/der Laboranten/Laborantin (14), auf der Prüfoberfläche (6, 106) eine seitliche Stellung einzunehmen, wobei dieser/diese Laborant/Laborantin (14) seine/ihre Füße in einer zum Zugmittel (4) orthogonalen Richtung anordnet;
Veranlassen des/der Laboranten/Laborantin (14), ein Bein bezogen auf das Befestigungselement (2) nach hinten abzuspreizen, bis dieses Bein vollständig von der Prüfoberfläche (6, 106) angehoben ist, wobei der/die Laborant/Laborantin (14) auf dem verbleibenden Bein statisch das Gleichgewicht hält;
Veranlassen des/der Laboranten/Laborantin (14), sich seitlich in einer dem Befestigungselement (2) entgegengesetzten Richtung zu neigen, bis die Sohle (15) gegenüber der Prüfoberfläche (6, 106) Haftreibung einbüßt.

**16.** Verfahren nach einem der Ansprüche 11 bis 15, umfassend:

Sicherstellen, dass der/die Laborant/Laborantin (14) während der Ausführung der Haftreibungsprüfung richtig auf der Prüfoberfläche (6, 106) steht;
Sicherstellen, dass die Sohle (15) vollständig in Kontakt mit der Prüfoberfläche (6, 106) ist;
Sicherstellen, dass die Sohle (15) innerhalb eines Zeitraums in einem Bereich von zwei bis vier Sekunden ab Beginn der Haftreibungsprüfung Haftreibung gegenüber der Prüfoberfläche (6, 106) einbüßt.

**17.** Verfahren nach einem der Ansprüche 11 bis 16, umfassend:

Analysieren der von der Messeinrichtung (3) während den Haftreibungsprüfungen bereitgestellten Daten, um die Griffigkeit der Schuhsohle (15) gegenüber den Prüfoberflächen (6, 106) zu prüfen.

**Revendications**

**1.** Dispositif (1) pour effectuer des tests d'adhérence d'une semelle (15) pour chaussures, lesdites chaussures avec ladite semelle de chaussures (15) à tester étant portées par un opérateur (14), ledit dispositif (1) comprenant au moins un élément de fixation (2), un dispositif de mesure (3) connecté audit élément de fixation (2), des moyens de maintien (5) associables audit opérateur (14) qui porte lesdites chaussures, un moyen de traction (4) fixé à une première extrémité audit dispositif de mesure (3) et à une deuxième extrémité auxdits moyens de maintien (5), **caractérisé en ce qu'**il comprend une surface de test (6, 106) pour effectuer un test d'adhérence de ladite semelle de chaussures (15), ledit opérateur (14) pouvant être positionné sur ladite surface de test (6, 106).

2. Dispositif selon la revendication 1, dans lequel ledit élément de fixation (2) est maintenu immobile par rapport à ladite surface de test (6, 106), par exemple en étant fixé, à une extrémité de celui-ci, à un sol (8) ou à une paroi verticale (9).

3. Dispositif selon la revendication 1 ou 2, dans lequel ledit élément de fixation (2) comprend un oeillet (7) pour être connecté audit dispositif de mesure (3).

4. Dispositif selon l'une quelconque des revendications précédentes, dans lequel ledit dispositif de mesure (3) comprend un dynamomètre électronique équipé d'un capteur de force et/ou un dynamomètre mécanique.

5. Dispositif selon l'une quelconque des revendications précédentes, dans lequel ledit moyen de traction (4) comprend un élément de corde et/ou un élément rigide ou flexible adapté à cet effet.

6. Dispositif selon l'une quelconque des revendications précédentes, dans lequel ledit moyen de traction (4) a une longueur variable.

7. Dispositif selon l'une quelconque des revendications précédentes, dans lequel lesdits moyens de maintien (5) comprennent un harnais de sécurité (10) et au moins un élément de connexion (11), constitué par un anneau et/ou un mousqueton et/ou un élément similaire, pour connecter ledit harnais (10) audit moyen de traction (4).

8. Dispositif selon l'une quelconque des revendications précédentes, dans lequel ladite surface de test (6, 106) est placée sur un châssis horizontal (13, 113) fixé immobile au sol.

9. Dispositif selon l'une quelconque des revendications précédentes, dans lequel ladite surface de test (6) a un profil sensiblement semi-circulaire et/ou circulaire.

10. Dispositif selon l'une quelconque des revendications précédentes 1 à 8, dans lequel ladite surface de test (6) a un profil sensiblement semi-circulaire ou circulaire et comprend plusieurs zones (12) disposées radialement l'une après l'autre, chacune ayant ses propres caractéristiques telles que la rugosité de surface et/ou le type de matériau et/ou la finition de surface, etc.

11. Procédé pour effectuer un test d'adhérence sur des semelles de chaussures comprenant les étapes suivantes:

   la fourniture d'un dispositif (1, 101) comprenant au moins un élément de fixation (2), un dispositif de mesure (3), un moyen (5) pour maintenir un opérateur (14), et au moins une surface de test (6, 106) **caractérisé en ce qu'** il comprend les étapes suivantes:

   la préparation de ladite surface de test (6, 106) et d'une semelle de chaussures (15) à tester;
   l'exécution d'un test d'adhérence desdites semelles de chaussures (15) sur au moins une de ladite surface de test (6, 106) du dispositif (1), lesdites chaussures, pourvues de ladite semelle de chaussures (15), étant portées par ledit opérateur (14);
   l'enregistrement de paramètres dudit test d'adhérence et de données fournies par ledit dispositif de mesure (3).

12. Procédé selon la revendication 11, comprenant la pulvérisation de ladite surface de test (6, 106) avec différentes substances, par exemple de l'eau et/ou des huiles et/ou des paraffines, etc.

13. Procédé selon la revendication 11 ou 12, comprenant le nettoyage de ladite surface de test (6, 106) avec de l'eau, par exemple de l'eau courante et/ou une solution d'éthanol.

14. Procédé selon l'une quelconque des revendications 11 à 13, comprenant le nettoyage desdites semelles à tester avec une solution d'éthanol et d'eau et/ou par abrasion exécutée avec du papier abrasif sans modifier sensiblement lesdites semelles, en particulier une éventuelle semelle extérieure desdites semelles, et/ou par des jets d'air comprimé ou des brosses douces.

15. Procédé selon l'une quelconque des revendications 11 à 14, comprenant:

   faire prendre audit opérateur (14) une position latérale sur ladite surface de test (6, 106), ledit opérateur (14) plaçant ses pieds dans une direction orthogonale par rapport audit moyen de traction (4);
   faire plier une jambe audit opérateur (14), ladite jambe étant placée en arrière par rapport audit élément de fixation (2), jusqu'à soulever complètement ladite jambe de ladite surface de test (6, 106), ledit opérateur (14) s'équilibrant d'une manière statique sur ladite jambe d'appui;
faire plier ledit opérateur (14) latéralement dans une direction opposée audit élément de fixation (2), jusqu'à ce que ladite semelle (15) perde l'adhérence par rapport à ladite surface de test (6, 106).

16. Procédé selon l'une quelconque des revendications 11 to 15, comprenant:

s'assurer que ledit opérateur (14) est correctement placé sur ladite surface de test (6, 106) durant l'exécution dudit test d'adhérence;
s'assurer que ladite semelle (15) est placée complètement en contact avec ladite surface de test (6, 106);
s'assurer que ladite semelle (15) perde l'adhérence par rapport à ladite surface de test (6, 106) dans une période de temps allant de deux à quatre secondes à compter du début dudit test d'adhérence.

17. Procédé selon l'une quelconque des revendications 11 à 16, comprenant:

l'analyse de données fournies par ledit Dispositif de mesure (3), durant lesdits tests d'adhérence, de manière à vérifier la puissance d'adhérence de ladite semelle de chaussures (15) par rapport auxdites surfaces de test (6, 106).

FIG.1

FIG.2

FIG.3

FIG.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4187714 A
- WO 2009077733 A